## Europäisches Patentamt
## European Patent Office
## Office européen des brevets

(19)

(11) Numéro de publication: **0 184 662**
**B1**

(12) # FASCICULE DE BREVET EUROPÉEN

(45) Date de publication du fascicule du brevet:
**12.07.89**

(51) Int. Cl.⁴: **A 61 L 9/12, A 45 D 34/00**

(21) Numéro de dépôt: **85114034.3**

(22) Date de dépôt: **05.11.85**

(54) **Diffuseur de parfum.**

(30) Priorité: **10.11.84 CH 5376/84**

(43) Date de publication de la demande:
**18.06.86 Bulletin 86/25**

(45) Mention de la délivrance du brevet:
**12.07.89 Bulletin 89/28**

(84) Etats contractants désignés:
**AT BE DE FR GB IT LU NL SE**

(56) Documents cité:
**FR-A-961 219**
**FR-A-1 243 273**
**US-A-4 243 159**

(73) Titulaire: **ANCIENNE MAISON SANDOZ FILS & CIE SA, Avenue Léopold- Robert 104- 106, CH- 2300 La Chaux de Fonds (CH)**

(72) Inventeur: **Reyna, Alain, Avenue Léopold- Robert 57, CH- 2300 La Chaux de Fonds (CH)**

(74) Mandataire: **Gresset, Jean, c/o SOPRINTEL SA 42, Avenue Léopold- Robert, CH- 2300 La Chaux de Fonds (CH)**

EP 0 184 662 B1

## Description

La présente invention se rapporte aux diffuseurs de parfum. Elle concerne plus particulièrement un diffuseur laissant échapper de façon continue les vapeurs émises par un parfum contenu dans un réservoir.

Dans les diffuseurs de ce type actuellement connus, la réserve de parfum est sous forme solide. Il s'agit généralement d'une cire parfumée. L'inconvénient de tels systèmes est qu'ils ne sont pas rechargeables. Ils deviennent donc inutiles lorsque la réserve de produit parfumé est épuisee.

La présente invention a pour but de pallier l'inconvénient des systèmes connus, en fournissant un diffuseur de parfum qui utilise une réserve de parfum liquide et peut être rechargé facilement et rapidement.

Pour atteindre ce but, le diffuseur selon l'invention comporte un récipient comprenant une ouverture et capable de recevoir du parfum liquide, un bouchon obturant ladite ouverture et percé d'un premier canal reliant l'intérieur du récipient à l'extérieur et d'un deuxième canal reliant le premier canal à l'extérieur, un tube plongeant à l'intérieur du récipient et dont la partie supérieure prend place dans ledit premier canal à l'intérieur duquel elle est libre de coulisser, une butée solidaire dudit tube et destinée à définir la position de repos de celui-ci en venant au contact de la face interne dudit bouchon, et un ressort agissant sur ladite butée pour l'appliquer contre ladite face interne de manière à obturer de façon étanche ledit premier canal.

D'autres caractéristiques de l'invention, ainsi que ses avantages, ressortiront de la description qui va suivre, faite en regard des dessins annexés et donnant, à titre explicatif mais nullement limitatif, une forme de réalisation avantageuse de ce diffuseur.

Sur ces dessins, où les mêmes numéros de référence se rapportent aux mêmes éléments:

- la figure 1 représente un diffuseur selon l'invention; et
- la figure 2 représente ce même diffuseur lors de son remplissage.

Comme on le voit sur les figures, le diffuseur 10 comporte un récipient cylindrique 11 avantageusement réalisé en un matériau transparent, tel que le "plexiglas". Le récipient comporte, à sa partie supérieure, une ouverture circulaire obturable par un bouchon 12 comportant une partie supérieure 12a, de diamètre sensiblement égal au diamètre extérieur du récipient, qui repose par sa périphérie sur le bord de celui-ci, et une partie inférieure 12b, de diamètre égal au diamètre intérieur du récipient, qui est collée ou engagée à force dans ce dernier de manière à réaliser l'étanchéité.

Le bouchon 12 comporte, en son centre, un canal circulaire 13 dans lequel peut coulisser avec un jeu notable la partie supérieure d'un tube 14.

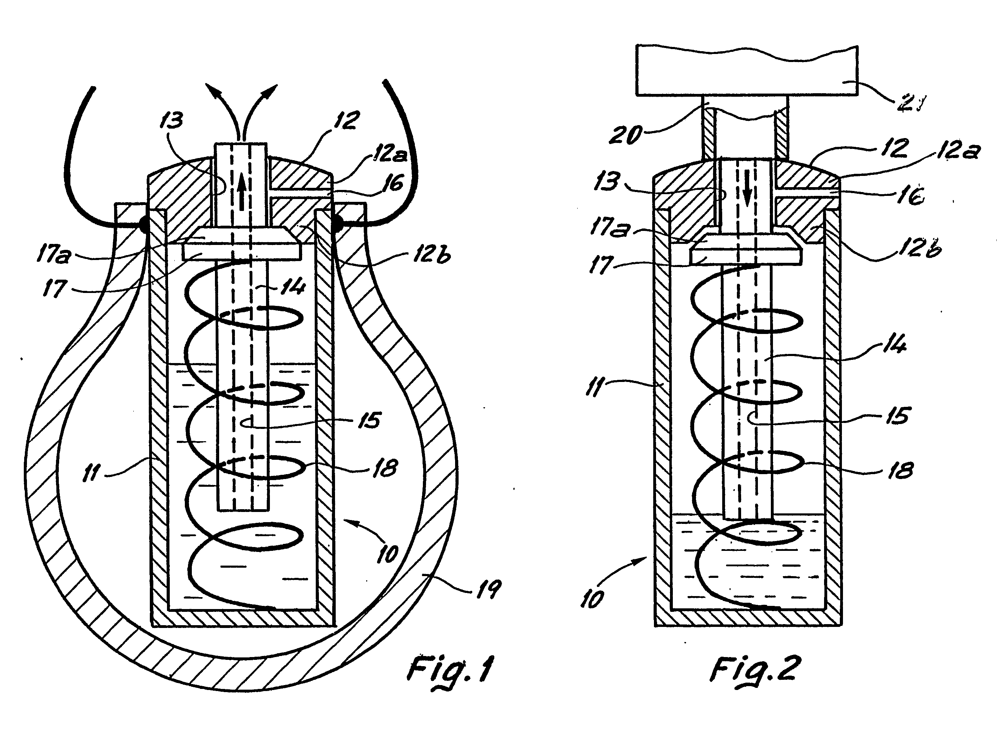

Lorsque celui-ci est dans sa position de repos (représentée à la figure 1), qui correspond à la position de fonctionnement normal du diffuseur, son extrémité dépasse légèrement la face supérieure du bouchon 12. La raison de cette particularité apparaîtra plus loin dans la description. Le tube s'étend sur la plus grande partie de la longueur du récipient 11 sans toutefois atteindre son fond. Le conduit central de ce tube est désigné par la référence 15.

La partie supérieure 12a du bouchon est percée d'un canal radial 16 reliant à l'extérieur le canal central 13.

Le tube 14 porte, à sa partie supérieure, une butée cylindrique 17, de diamètre sensiblement inférieur à celui du récipient, ayant pour double rôle de définir la position de repos du tube et d'obturer alors de façon étanche le canal 13. Sa partie supérieure 17a, tronconique, prend place dans un évidement de forme complémentaire ménagé dans la face interne du bouchon 12. Pour assurer l'étanchéité, cette butée est maintenue appliquée contre le bouchon sous l'action d'un ressort à boudin 18, entourant le tube, qui prend appui entre la butée et le fond du récipient. Bien entendu, il n'est pas nécessaire de faire descendre le ressort 18 jusqu'au fond du récipient 11. Dans ce cas, le ressort peut simplement prendre appui sur un épaulement ménagé dans la paroi interne du réservoir.

Il va de soi que tout autre moyen de rappel de la butée en position de repos peut être utilisé, comme par exemple une lame ou une rondelle de matière élastique, telle que de l'acier à ressort, du bronze au béryllium ou du plastique.

Le bouchon 12, le tube 14 et la butée 17 sont avantageusement réalisés en laiton. Ils peuvent cependant être réalisés en toute autre matière, du plastique aux matériaux nobles, tel que l'or. Il en est de même pour le ressort. Par ailleurs, le tube et la butée forment, de préférence, une pièce unique.

Les dimensions du récipient 11, et même sa forme, dépendent essentiellement de sa destination. La conception du diffuseur selon l'invention permet cependant de le réaliser en très petites dimensions afin de le placer dans un article de bijouterie. La figure 1 montre, à titre d'exemple, le diffuseur 10 disposé à l'intérieur d'un bijou 19, en forme de vase, monté en pendentif. Il est même possible, et souvent très avantageux, de faire jouer à l'article en question le rôle de réservoir de parfum. Ainsi, dans le cas de la figure 1, le récipient 11 est supprimé et le bouchon 12 inséré directement (par vissage ou collage) dans son ouverture. Une telle solution permet d'augmenter la capacité du réservoir de parfum et de réduire sensiblement le prix de revient du système.

Si les dimensions du récipient 11 peuvent être quelconques, dans une forme de réalisation avantageuse de l'invention, il n'en est pas tout à fait de même pour celles du tube 14. En effet, le diffuseur de parfum selon l'invention est conçu pour être rempli à l'aide d'une bombe de recharge usuelle contenant un parfum liquide sous pression. Il importe donc que le diamètre du tube soit compatible avec les dimensions des buses des bombes disponibles sur le marché. Le remplissage peut aussi, bien entendu, être effectué à l'aide d'une seringue ou d'une capsule en matière souple faisant office de seringue. Dans ce cas, les dimensions du tube sont sans importance.

A titre indicatif, pour des applications à l'horlogerie, la bijouterie, la parfumerie ou pour les bibelots, le tube 14 a un diamètre externe de l'ordre de 0,50 à 5,00 mm. Le conduit central 15 du tube a, typiquement, un diamètre de 0,20 à 1,00 mm. Enfin, le diamètre du canal radial 16 du bouchon a un diamètre de 0,20 à 0,30 mm.

On se reportera maintenant à la figure 2 pour décrire l'opération de remplissage du diffuseur selon l'invention. L'extrémité de la buse 20 d'une bombe de recharge 21 étant appliquée contre le bouchon 12, en son centre, le parfum sous pression qu'elle libère provoque un léger enfoncement du tube 14. La butée 17 sort donc de son siège, de sorte que la sortie du canal 13 n'est plus obturée de façon étanche. Le parfum pénètre par le conduit central 15 du tube dans le réservoir et l'air contenu dans celui-ci est évacué par le canal radial 16 après avoir emprunté l'espace libre, dû au jeu déjà mentionné, entre le tube 14 et la paroi interne du canal 13.

Au fur et à mesure que le récipient 11 se remplit de liquide, celui-ci exerce sur la partie immergée du tube 14 une poussée croissante vers le haut qui, ajoutée à l'action du ressort 18, finit par contre-carrer la poussée qu'exerce en sens inverse le liquide éjecté de la bombe 21, de sorte que la butée 17 revient obturer de façon étanche le canal 13. Très rapidement, vu que l'air emprisonné dans le réservoir ne peut donc plus s'échapper, la pression à l'intérieur de celui-ci s'oppose à toute nouvelle introduction de parfum.

A ce moment, qui intervient donc avant que le réservoir soit plein, le liquide qui sort de la bombe emprunte l'espace libre entre le tube 14 et la paroi du canal 13 et s'échappe du canal radial sous forme d'un jet. Ceci indique que l'opération de recharge est terminée.

Dès lors, le parfum liquide emprisonné dans le réservoir peut s'évaporer lentement et de façon constante par le conduit central 15 du tube.

Il est important de noter qu'en cas de retournement du diffuseur, étant donné que le récipient 11 n'est pas complètement rempli et donc que le niveau du liquide se trouve en dessous de l'extrémité du tube 14, le parfum ne risque pas de s'échapper.

Bien entendu, si l'on souhaite obtenir quelques gouttes du parfum sous forme liquide, il suffit de retourner le diffuseur et d'appuyer sur le tube 14 qui, comme déjà indiqué, dépasse le bouchon 12. Ce mouvement permet de faire sortir la butée 17 de son siège, qui laisse-ainsi du liquide s'écouler entre le tube 14 et le canal 13.

Ainsi, est réalisé un diffuseur de parfum utilisant une réserve de parfum liquide et pouvant être rechargé facilement et rapidement à l'aide d'une bombe de recharge usuelle. Le but de l'invention est donc atteint.

De plus, ce diffuseur se prête aisément à la miniaturisation. Il est donc très bien adapté pour être utilisé dans des articles de bijouterie. Enfin, l'obtention, à la demande, de quelques gouttes de parfum est particulièrement aisée.

## Revendications

1. Diffuseur pour laisser échapper de façon continue les vapeurs émises par parfum, caractérisé en ce qu'il comporte:
- un récipient comprenant une ouverture et capable de recevoir ledit parfum sous forme liquide;
- un bouchon obturant ladite ouverture et percé d'un premier canal reliant l'intérieur du récipient à l'extérieur et d'un deuxième canal reliant le premier canal à l'extérieur;
- un tube plongeant à l'intérieur du récipient et dont la partie supérieure prend place dans ledit premier canal, à l'antérieur duquel elle est libre de coulisser;
- une butée solidaire dudit tube et destinée à définir la position de repos de celui-ci en venant au contact de la face interne dudit bouchon; et
- un ressort agissant sur ladite butée pour l'appliquer contre ladite face interne de manière à obturer de façon étanche ledit premier canal.

2. Diffuseur selon la revendication 1, caractérisé en ce que la face inférieure du bouchon comporte un évidement dans lequel prend place la partie supérieure de la butée lorsque le tube est en position de repos.

3. Diffuseur selon l'une des revendications 1 et 2, caractérisé en ce que le tube et la butée forment une pièce unique.

4. Diffuseur selon l'une des revendications précédentes, caractérisé en ce que ledit ressort est un ressort à boudin entourant le tube.

5. Diffuseur selon l'une des revendications précédentes, caractérisé en ce que, lorsque le tube est en position de repos, son extrémité supérieure dépasse la face supérieure du bouchon.

## Patentansprüche

1. Diffusor zum kontinuierlichen Ausstossen der von einem Parfüm freigesetzten Dämpfe, dadurch gekennzeichnet, dass er folgende Bestandteile umfasst:
- einen Behälter mit einer Öffnung, der das genannte Parfüm in flüssiger Form aufnehmen kann;
- einen Verschluss, der die genannte Öffnung abdeckt und von einem ersten Kanal durchbohrt ist, der die Innenseite des Behälters mit der Aussenseite verbindet, und von einem zweiten Kanal, der den ersten Kanal mit der Aussenseite verbindet;

- ein in die Innenseite des Behälters eintauchendes Rohr, dessen oberer Teil im genannten ersten Kanal angeordnet ist, innerhalb dessen es sich frei verschieben kann;

- einen mit dem genannten Rohr fest verbundenen Anschlag, der dessen Ruhestellung definiert, indem er sich an der Innenfläche des genannten Verschlusses abstützt; und

- eine Feder, die auf den genannten Anschlag wirkt, um diesen gegen die genannte Innenfläche so anzudrücken, dass der genannte erste Kanal dicht verschlossen wird.

2. Diffusor gemäss Anspruch 1, dadurch gekennzeichnet, dass die Innenfläche des Verschlusses eine Aussparung umfasst, in der sich der obere Teil des Anschlags einfügt, wenn sich das Rohr in Ruhestellung befindet.

3. Diffusor gemäss einem der Ansprüche 1 und 2, dadurch gekennzeichnet, dass das Rohr und der Anschlag ein einziges Teil bilden.

4. Diffusor gemäss einem der vorstehenden Ansprüche, dadurch gekennzeichnet, dass die genannte Feder eine das Rohr umgebende Schraubenfeder ist.

5. Diffusor gemäss einem der vorstehenden Ansprüche, dadurch gekennzeichnet, dass sein oberes Ende die obere Fläche des Verschlusses überragt, wenn sich das Rohr in Ruhestellung befindet.

**Claims**

1. A diffusing device for letting out in a continuous manner the vapours given off by a perfume, characterized in that it comprises:

- a container having an opening and able to receive said perfume in liquid form;

- a stopper closing off said opening and formed with a first channel that connects the inside of the container to the outside and with a second channel that connects the first channel to the outside;

- a tube that dips into the interior of the container and whose upper portion is housed in said first channel, inside which it is free to slide;

- an abutment solid with said tube and intended to define the rest position of the latter by engaging the inner surface of said stopper; and

- a spring acting on said abutment to apply it against said inner surface such as to close off hermetically said first channel.

2. A diffusing device according to claim 1, characterised in that the lower surface of the stopper has a recess in which is housed the upper portion of the abutment when the tube is in its rest position.

3. A diffusing device according to either of claims 1 and 2, characterized in that the tube and the abutment are made of one piece.

4. A diffusing device according to any preceding claim, characterised in that said spring is a coil spring surrounding the tube.

5. A diffusing device according to any preceding claim, characterised in that, when the tube is in its rest position, its top end projects above the upper surface of the stopper.

Fig.1

Fig.2

EP 0 184 662 B1